# EUROPEAN PATENT APPLICATION

(11) **EP 2 074 948 A1**
(43) Date of publication of application: **01.07.2009**
(21) Application number: 08022484.3
(22) Date of filing: 29.12.2008
(51) Int. Cl.: A61B 8/08

(54) **Ultrasound system for diagnosing breast cancer**

(30) Priority: 27.12.2007 KR 20070138430; 23.12.2008 KR 20080132601
(71) Applicant: MEDISON CO., LTD., Kangwon-do 250-870 (KR)
(72) Inventor: Kim, Jeong Hwan, Gangnam-gu, Seoul 135-280 (KR); Chang, Seomg Ho, Gangnam-gu, Seoul 135-280 (KR)
(74) Representative: Raunecker, Klaus Peter

(57) **Abstract**

The present invention relates to an ultrasound system for diagnosing breast cancers. The ultrasound system comprises an ultrasound probe including an array transducer having an arcuate shape, the array transducer containing a plurality of elements arrayed in a longitudinal direction of the array transducer. The ultrasound probe further includes a probing surface for contacting a surface of the target object, wherein the probing surface is formed to have a concave shape at a front side of the array transducer.

## Description

The present application claims priority from Korean Patent Application Nos. 10-2007-0138430 and 10-2008-0132601 filed on December 27, 2007 and December 23, 2008, the entire subject matters of which are incorporated herein by references.

### BACKGROUND OF THE INVENTION

### [Technical Field]

The present invention generally relates to ultrasound systems, and more particularly to an ultrasound system adopting an ultrasound probe for diagnosing breast cancers.

### [Background Art]

Breast cancers are a significant medical concern for women. The breast cancers significantly increase the mortality rate of women and also lead to severe psychological/emotional trauma as well as economic loss. Thus, preventing breast tumors and conducting regular periodic medical examinations to find such tumors at an early stage are very important. As such, there has been introduced a breast cancer diagnostic apparatus that uses X-rays for performing early diagnosis of breast tumors.

However, since the conventional breast cancer diagnostic apparatus may press the patient's breast with about 20 kg load, the patient feels severe pain. Also, it may be difficult for the patient to maintain a stationary posture due to such pain. As such, accurate images may not be obtained.

Recently, in order to address and resolve the above-mentioned problems, an ultrasound system has been used to provide elastic images for diagnosing breast tumors based on the stress applied through a probe. However, the patent still feels much pain since the elastic images are provided while pressing the breast.

### BRIEF DESCRIPTION OF THE DRAWINGS

FIG. 1 is a perspective view showing one embodiment of an ultrasound probe.

FIG. 2 is a cross-sectional view showing one embodiment of an ultrasound probe.

FIG. 3 is a schematic diagram showing an example of rotating an array transducer.

FIG. 4 is a block diagram showing an illustrative embodiment of an ultrasound system with an ultrasound probe having an array transducer with an arcuate shape.

FIG. 5 is a schematic diagram showing an example of scan lines originated from elements of an array transducer.

### DETAILED DESCRIPTION OF THE INVENTION

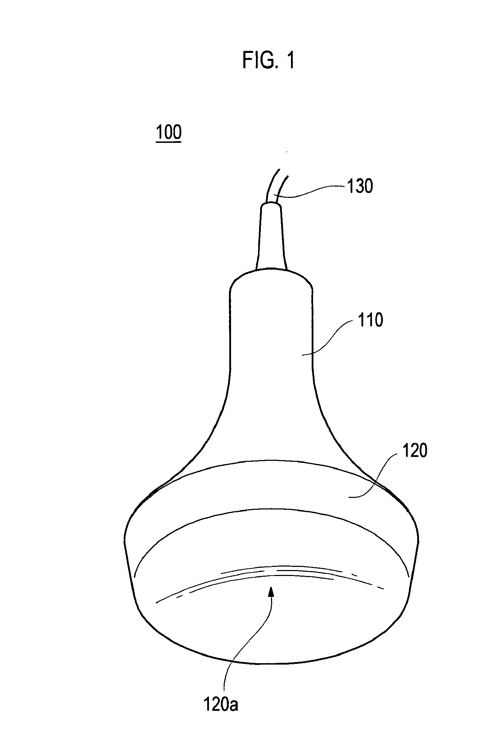

FIG. 1 is a perspective view showing one embodiment of an ultrasound probe. As shown in FIG. 1, the ultrasound probe 100 may include a grip portion 110, an ultrasound transmitting/receiving portion 120 and a cable 130 connected to a body (not shown). The grip portion 110 may be formed to have a trumpet shape. This is so that an easy and stable grip may be provided and an end of the grip portion 110 may be coupled to the ultrasound transmitting/receiving portion 120. However, the grip portion 110 may not be limited to such a shape. That is, the grip portion 110 may be formed in various shapes according to necessity. The ultrasound transmitting/receiving portion 120 may be formed to have a cylindrical shape. The ultrasound transmitting/receiving portion 120 may include a probing surface 120a of a concave shape at a bottom side thereof for contacting the surface of a target object.

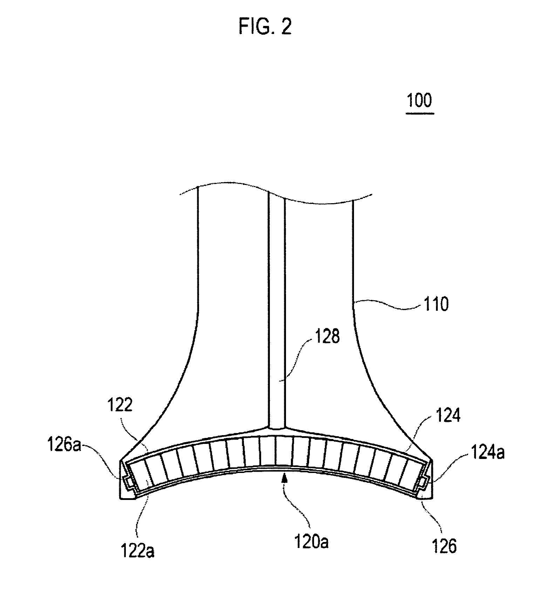

FIG. 2 is a cross-sectional view schematically showing one embodiment of the ultrasound probe 100. The ultrasound transmitting/receiving portion 120 may include an array transducer 122. The array transducer 122 may include a plurality of elements 122a, which are arrayed in a longitudinal direction to have an arcuate shape. A front side of the array transducer 122, i.e., a concave portion thereof, may face the probing surface 120a. Lubricant oil may be filled between the array transducer 122 and the probing surface 120a. The plurality of elements 122a may be configured to generate ultrasound signals. Also, the elements 122a may further convert ultrasound signals reflected from the target object into electrical receive signals. The ultrasound transmitting/receiving portion 120 may further include a supporting part 124 surrounding the array transducer 122 for support. The support part 124 may include projecting parts 124a at both end sides of the array transducer 122. The projecting parts 124a may be formed to have various shapes such as a rectangle, triangle, trapezoid and the like. The supporting part 124 may further include a backing layer for protecting the ultrasound signals generated from the elements 122a from being transmitted backward.

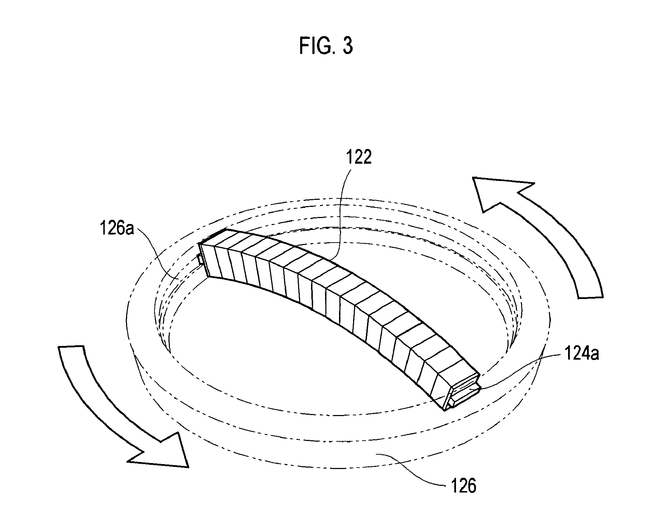

The ultrasound transmitting/receiving portion 120 may further include a guiding part 126 for guiding the array transducer 122. The guiding part 126 may be formed to have a circular shape. The guiding part 126 may include a guide rail 126a formed in a circumference direction at an inner surface of the guiding part 126 for receiving the projecting parts 124a and guiding the movements of the projecting parts 124a. The ultrasound transmitting/receiving portion 120 may further include a driving motor (not shown) and a rotating shaft 128 connected to the driving motor. The rotating shaft 128 may be fixed to a center of the array transducer 122 by the supporting part 124. The rotating shaft 128 may transfer a power of the motor to the array transducer 122 such that the array transducer 122 is rotated in the guiding part 126 along the guide rail 126a, as shown in FIG. 3. The array transducer 122 may transmit the ultrasound signals while the probing surface 120a is in contact with the surface of the target object (especially the breast).

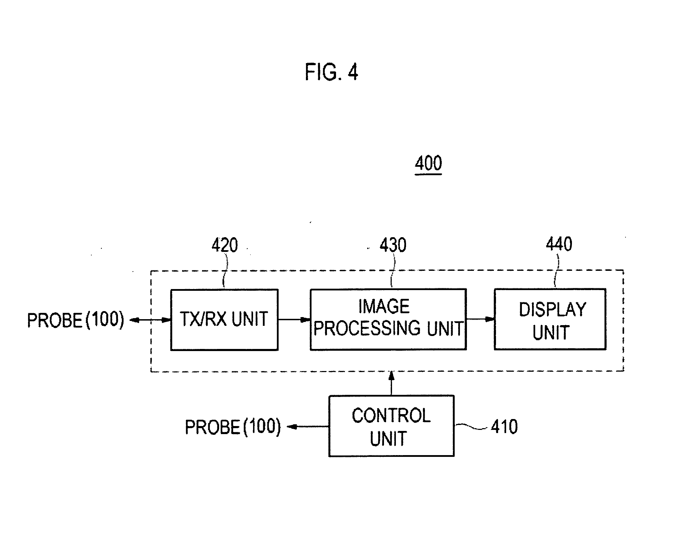

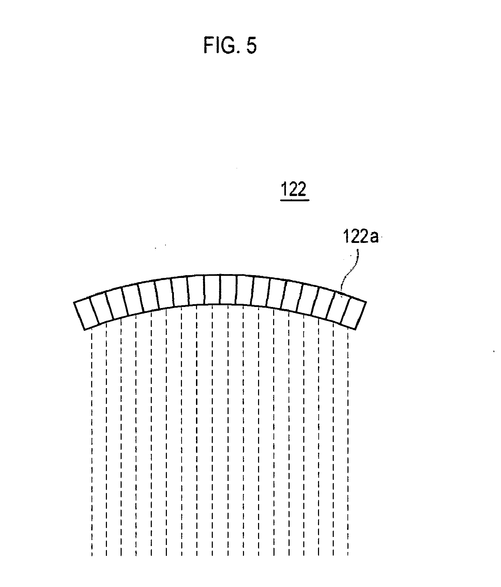

FIG. 4 is a block diagram showing an ultrasound system adopting one embodiment of the ultrasound probe. The ultrasound system 400 may include a control unit 410, a transmission/reception unit 420, an image processing unit 430 and a display unit 440. The control unit 410 may be configured to generate control signals for controlling the driving motor in the ultrasound probe 110 for rotating the array transducer 122 at a predetermined angle range as well as the transmission/reception of the ultrasound signals at the elements 122a of the array transducer 122. In one embodiment, the control unit 410 may generate a first control signal for controlling the driving motor such that the array transducer 122 may be rotated within 180 degrees. The control unit 410 may generate a second control signal for the transmission and reception of the ultrasound signals. Since the array transducer 122 is formed to have an arcuate shape in one embodiment, the control unit 410 control angles of scan lines originated from the elements 122a such that the scan lines are set in parallel, as illustrated in FIG. 5.

The transmission/reception unit 420 may be configured to generate transmit pulse signals in consideration of distances between the respective elements 122a and focal points set on the scan lines in the target object in response to the second control signal. The transmit pulse signals may be applied to the elements 122a through the cable 130. Also, the transmission/reception unit 420 may perform receive focusing upon receive signals outputted from the array transducer 122 in consideration of distances between the respective elements 122a and the focal points, thereby outputting receive-focused beams. The receive focused beams may be obtained while the array transducer 122 is rotated.

The image processing unit 430 may be configured to perform image processing upon the focused beams to form a 3-dimensional ultrasound image. The display unit 440 may display the 3-dimensional ultrasound image of the target object.

As the probing surface 120a and the array transducer 122 in the ultrasound probe 100 are formed to have an arcuate shape, a breast image may be obtained without feeling pain caused by pressure. Also, since the ultrasound signals are transmitted and received while the array transducer is rotated at a predetermined angle range in a horizontal direction to the surface of the target object, a 3-dimensional ultrasound image may be provided.

In accordance with one embodiment of the present invention, there is provided an ultrasound system, comprising: an ultrasound probe including an array transducer having an arcuate shape, the array transducer containing a plurality of elements arrayed in a longitudinal direction of the array transducer; and a probing surface for contacting a surface of the target object, wherein the probing surface is formed to have a concave shape at a front side of the array transducer.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound probe including an array transducer having an arcuate shape, the array transducer containing a plurality of elements arrayed in a longitudinal direction of the array transducer, the ultrasound probe further including a probing surface for contacting a surface of a target object, wherein the probing surface is formed to have a concave shape at a front side of the array transducer.

2. The ultrasound system of Claim 1, wherein the ultrasound probe further includes:
a guiding part having a cylindrical shape for guiding a rotation of the array transducer in a circumference direction at an inter surface of the guiding part ; and
a driving unit configured to rotate the array transducer in the guiding part.

3. The ultrasound system of Claim 2, wherein the driving unit includes:
a driving motor configured to provide a power; and
a rotating shaft connected to the driving motor and the array transducer for transferring the power to the array transducer for the rotation.

4. The ultrasound system of Claim 3, further comprising:
a control unit configured to generate a first control signal for controlling transmission/reception of ultrasound signals at the array transducer and a second control signal for controlling the driving motor for the rotation of the array transducer;
a transmission/reception unit configured to generate transmit pulse signals in consideration of distances between the elements and focal points and perform receive-focusing the receive signals to thereby output receive-focused beams; and
an image processing unit configured to form a 3-dimensional image based on the receive focused beams.

5. The ultrasound system of Claim 4, wherein the control unit is further configured to control angles of scan lines originated from the elements.
